(19) 

**Europäisches Patentamt**
**European Patent Office**
**Office européen des brevets**

(11) **EP 4 616 807 A1**

(12) **EUROPEAN PATENT APPLICATION**

(43) Date of publication:
 **17.09.2025 Bulletin 2025/38**

(21) Application number: **25162809.5**

(22) Date of filing: **11.03.2025**

(51) International Patent Classification (IPC):
 **A61B 6/00** *(2024.01)* **A61B 6/50** *(2024.01)*
 **G01N 23/04** *(2018.01)*

(52) Cooperative Patent Classification (CPC):
 **A61B 6/482; A61B 6/505; A61B 6/5205;**
 **A61B 6/5217; G01N 23/04;** A61B 6/544

(84) Designated Contracting States:
 **AL AT BE BG CH CY CZ DE DK EE ES FI FR GB**
 **GR HR HU IE IS IT LI LT LU LV MC ME MK MT NL**
 **NO PL PT RO RS SE SI SK SM TR**
 Designated Extension States:
 **BA**
 Designated Validation States:
 **GE KH MA MD TN**

(30) Priority: **12.03.2024 JP 2024037899**

(71) Applicant: **FUJIFILM Corporation**
 **Tokyo 106-8620 (JP)**

(72) Inventor: **TAKAHASHI, Tomoyuki**
 **Kaisei-machi, 258-8538 (JP)**

(74) Representative: **Dehns Germany Partnerschaft**
 **mbB**
 **Theresienstraße 6-8**
 **80333 München (DE)**

(54) **RADIATION IMAGE PROCESSING DEVICE, RADIATION IMAGE PROCESSING METHOD, AND RADIATION IMAGE PROCESSING PROGRAM**

(57) A processor is configured to: acquire first to (n - 1)th (n ≥ 3) radiation images acquired by imaging a subject, which includes first to nth components each consisting of a single composition, with n - 1 types of radiation having different energy distributions; acquire a body thickness of the subject; derive thicknesses of the first to nth components by using the body thickness and the first to (n - 1)th radiation images; and derive first to nth component images in which the first to nth components are enhanced, respectively, based on the thicknesses of the first to nth components.

**FIG. 3**

RADIATION IMAGE PROCESSING DEVICE — 10

IMAGE ACQUISITION UNIT — 21

BODY THICKNESS ACQUISITION UNIT — 22

COMPONENT THICKNESS DERIVATION UNIT — 23

IMAGE DERIVATION UNIT — 24

DISPLAY CONTROLLER — 25

**Description**

BACKGROUND

Technical Field

The present disclosure relates to a radiation image processing device, a radiation image processing method, and a radiation image processing program.

Related Art

In the related art, energy subtraction processing using two radiation images obtained by irradiating a subject with two types of radiation having different energy distributions by using the fact that an attenuation amount of the transmitted radiation differs in accordance with the substance constituting the subject has been known. The energy subtraction processing is a method in which respective pixels of the two radiation images obtained as described above are associated with each other, and subtraction is performed after multiplying a weight coefficient based on an attenuation coefficient in accordance with a composition between pixels to acquire an image in which specific components, such as a bone part and a soft part, included in the radiation image are separated. In addition, a method of separating a soft tissue of the subject into fat and muscle by energy subtraction processing has also been proposed (see JP2023-047911A and JP2022-056084A).

In order to separate a plurality of components included in the subject by the energy subtraction processing, the attenuation coefficient of the soft part is required. The soft part does not consist of a single composition, and a plurality of components, such as fat and muscle, are complicatedly mixed. Also, the composition of the soft part varies greatly among individuals. Therefore, in a case in which the attenuation coefficient of the soft part is not obtained in accordance with a ratio between the fat and the muscle, it is not possible to accurately separate a plurality of components, such as the bone part and the soft part, in a case in which the processing is performed using the characteristics related to the attenuation of the radiation, such as the energy subtraction processing.

In this case, it is considered to separate the soft part into the fat and the muscle. However, in the energy subtraction processing, since two radiation images obtained by two types of radiation having different energy distributions are used, only radiation images of two components, such as the bone part and the soft part, can be obtained. That is, in a case in which n radiation images obtained by n types of radiation having different energy distributions are used, only n component images can be obtained.

SUMMARY OF THE INVENTION

The present disclosure has been made in view of the above-described circumstances, and an object of the present disclosure is to accurately separate n components of a subject included in a radiation image by using n radiation images obtained by n - 1 ($n \geq 3$) types of radiation having different energy distributions.

The present disclosure relates to a radiation image processing device comprising: at least one processor, in which the processor is configured to: acquire first to (n - 1)th ($n \geq 3$, n is a natural number) radiation images acquired by imaging a subject, which includes first to nth components each consisting of a single composition, with n - 1 types of radiation having different energy distributions; acquire a body thickness of the subject; derive thicknesses of the first to nth components by using the body thickness and the first to (n - 1)th radiation images; and derive first to nth component images in which the first to nth components are enhanced, respectively, based on the thicknesses of the first to nth components.

It should be noted that, in the radiation image processing device according to the present disclosure, the processor may be configured to acquire the body thickness by deriving the body thickness of the subject based on at least one of the first to (n - 1)th radiation images.

In addition, in the radiation image processing device according to the present disclosure, the processor may be configured to: acquire a first radiation image and a second radiation image that are acquired by imaging the subject with two types of radiation having different energy components; derive the body thickness of the subject based on at least one of the first radiation image or the second radiation image; derive thicknesses of a first component, a second component, and a third component of the subject by using the body thickness, the first radiation image, and the second radiation image; and derive a first component image, a second component image, and a third component image in which the first component, the second component, and the third component are enhanced, respectively, based on the thicknesses of the first component, the second component, and the third component.

In addition, in the radiation image processing device according to the present disclosure, the first component, the second component, and the third component may be fat, muscle, and a bone, respectively.

The present disclosure relates to a radiation image processing method executed by a computer, the radiation image processing method comprising: acquiring first to (n - 1)th ($n \geq 3$) radiation images acquired by imaging a subject, which includes first to nth components each consisting of a single composition, with n - 1 types of radiation having different energy distributions; acquiring a body thickness of the subject; deriving thicknesses of the first to nth components by using the body thickness and the first to (n - 1)th radiation images; and deriving first to nth component images in which the first to nth components are enhanced, respectively, based on the thicknesses of the first to nth components.

The present disclosure relates to a radiation image processing program causing a computer to execute: a procedure of acquiring first to (n - 1)th (n ≥ 3) radiation images acquired by imaging a subject, which includes first to nth components each consisting of a single composition, with n - 1 types of radiation having different energy distributions; a procedure of acquiring a body thickness of the subject; a procedure of deriving thicknesses of the first to nth components by using the body thickness and the first to (n - 1)th radiation images; and a procedure of deriving first to nth component images in which the first to nth components are enhanced, respectively, based on the thicknesses of the first to nth components.

According to the present disclosure, it is possible to accurately separate the n components of the subject included in the radiation image by using the n radiation images obtained by the n - 1 (n ≥ 3) types of radiation having different energy distributions.

BRIEF DESCRIPTION OF THE DRAWINGS

Fig. 1 is a schematic block diagram showing a configuration of a radiography system to which a radiation image processing device according to an embodiment of the present disclosure is applied.
Fig. 2 is a diagram showing a schematic configuration of the radiation image processing device according to the present embodiment.
Fig. 3 is a diagram showing a functional configuration of the radiation image processing device according to the present embodiment.
Fig. 4 is a diagram showing first and second radiation images.
Fig. 5 is a diagram showing an attenuation amount in accordance with a thickness of the muscle and a thickness of the fat in a high-energy image and a low-energy image.
Fig. 6 is a diagram showing a display screen.
Fig. 7 is a flowchart showing the processing performed in the present embodiment.

DETAILED DESCRIPTION

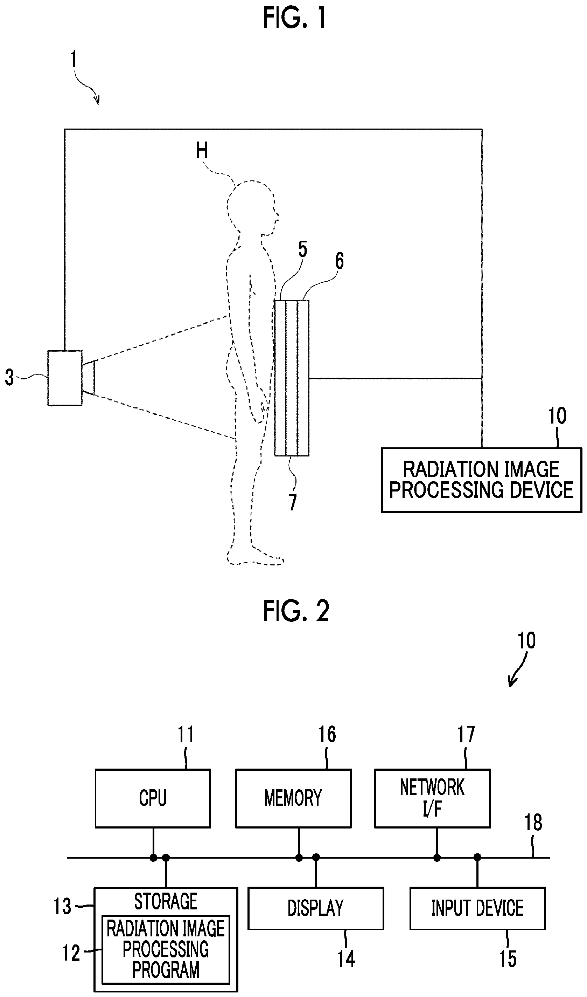

In the following description, an embodiment of the present disclosure will be described with reference to the drawings. Fig. 1 is a schematic block diagram showing a configuration of a radiography system to which a radiation image processing device according to the embodiment of the present disclosure is applied. As shown in Fig. 1, the radiography system according to the present embodiment comprises an imaging apparatus 1 and a radiation image processing device 10 according to the present embodiment.

The imaging apparatus 1 is an imaging apparatus for performing energy subtraction imaging by a so-called one-shot method for changing energy of each of radiation, such as X-rays, emitted from a radiation source 3 and transmitted through a subject H and irradiating a first radiation detector 5 and a second radiation detector 6 with the converted radiation. During the imaging, as shown in Fig. 1, the first radiation detector 5, a radiation energy conversion filter 7 made of a copper plate or the like, and the second radiation detector 6 are disposed in order from a side closest to the radiation source 3, and the radiation source 3 is driven. It should be noted that the first and second radiation detectors 5 and 6 are closely attached to the radiation energy conversion filter 7.

As a result, in the first radiation detector 5, a first radiation image G1 of the subject H by low-energy radiation also including so-called soft rays is acquired. In addition, in the second radiation detector 6, a second radiation image G2 of the subject H by high-energy radiation from which the soft rays are removed is acquired. The first and second radiation images G1 and G2 are input to the radiation image processing device 10.

The first and second radiation detectors 5 and 6 can perform recording and reading-out of the radiation image repeatedly, and a so-called direct-type radiation detector that directly receives emission of the radiation and generates an electric charge may be used, or a so-called indirect-type radiation detector that converts the radiation into visible light and then converts the visible light into an electric charge signal may be used. In addition, as a method for reading out a radiation image signal, it is desirable to use a so-called thin film transistor (TFT) readout method in which the radiation image signal is read out by turning a TFT switch on and off, or a so-called optical readout method in which the radiation image signal is read out by emission of read out light, but other methods may also be used without being limited to these methods.

Hereinafter, the radiation image processing device according to the present embodiment will be described. First, a hardware configuration of the radiation image processing device according to the present embodiment will be described with reference to Fig. 2. As shown in Fig. 2, the radiation image processing device 10 is a computer, such as a workstation, a server computer, and a personal computer, and comprises a central processing unit (CPU) 11, a nonvolatile storage 13, and a memory 16 as a transitory storage region.

In addition, the radiation image processing device 10 comprises a display 14, such as a liquid crystal display, an input device 15, such as a keyboard and a mouse, and a network interface (I/F) 17 connected to a network (not shown). The CPU 11, the storage 13, the display 14, the input device 15, the memory 16, and the network I/F 17 are connected to a bus 18. It should be noted that the CPU 11 is an example of a processor according to the present disclosure.

The storage 13 is implemented by a hard disk drive (HDD), a solid state drive (SSD), a flash memory, and the like. A radiation image processing program 12 installed in the radiation image processing device 10 is stored in the storage 13 as a storage medium. The CPU 11 reads out the radiation image processing program 12 from the storage 13, loads the readout radiation image processing program 12 to the memory 16, and executes the loaded radiation image processing program 12.

The radiation image processing program 12 is stored in a storage device of the server computer connected to the network or in a network storage in a state of being accessible from the outside, and is downloaded and installed in the computer that configures the radiation image processing device 10 in response to the request. Alternatively, the radiation image processing program 12 is distributed in a state of being recorded on a recording medium, such as a digital versatile disc (DVD) or a compact disc read only memory (CD-ROM), and is installed in the computer that configures the radiation image processing device 10 from the recording medium.

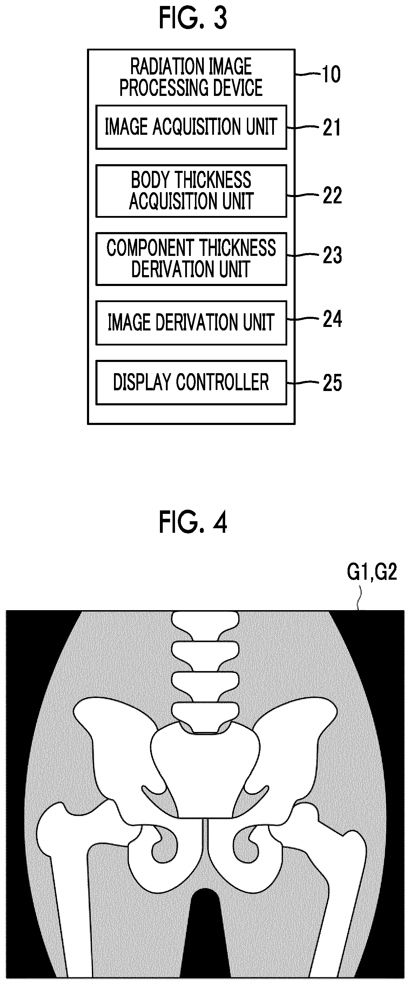

Next, a functional configuration of the radiation image processing device according to the present embodiment will be described. Fig. 3 is a diagram showing the functional configuration of the radiation image processing device according to the present embodiment. As shown in Fig. 3, the radiation image processing device 10 comprises an image acquisition unit 21, a body thickness acquisition unit 22, a component thickness derivation unit 23, an image derivation unit 24, and a display controller 25. Then, the CPU 11 executes the radiation image processing program 12 to function as the image acquisition unit 21, the body thickness acquisition unit 22, the component thickness derivation unit 23, the image derivation unit 24, and the display controller 25.

The image acquisition unit 21 acquires the first radiation image G1 and the second radiation image G2 of the subject H from the first and second radiation detectors 5 and 6 by causing the imaging apparatus 1 to perform the energy subtraction imaging of the subject H. In this case, imaging conditions, such as an imaging dose, an energy distribution, a tube voltage, and an SID, are set. The imaging conditions need only be set by input from the input device 15 by a user. The set imaging conditions are stored in the storage 13. It should be noted that the first and second radiation images G1 and G2 may be acquired by a program different from the radiation image processing program according to the present embodiment. In this case, the image acquisition unit 21 reads out the first and second radiation images G1 and G2 stored in the storage 13 from the storage 13 for processing.

Fig. 4 is a diagram showing the first and second radiation images. As shown in Fig. 4, a region of the subject H and a direct radiation region obtained by directly irradiating the radiation detectors 5 and 6 with the radiation are included in the first and second radiation images G1 and G2. A soft region and a bone region are included in the region of the subject H. A soft part component of a human body includes muscle, fat, blood, and water. In the present embodiment, a non-fat tissue including blood and water is treated as the muscle. The fat component, the muscle component, and the bone part component of the subject H are examples of a first component, a second component, and a third component according to the present disclosure, respectively.

The body thickness acquisition unit 22 acquires the body thickness by deriving the body thickness of the subject H for each pixel of the first and second radiation images G1 and G2 based on at least one of the first radiation image G1 or the second radiation image G2. Here, the body thickness is a thickness in a direction in which the radiation is transmitted through the subject H, that is, a thickness in a direction perpendicular to the first and second radiation images G1 and G2. Since the body thickness is derived for each pixel of the first and second radiation images G1 and G2, the body thickness acquisition unit 22 derives a body thickness distribution in at least one of the first radiation image G1 or the second radiation image G2. In the derivation of the body thickness, the body thickness acquisition unit 22 uses the first radiation image G1 acquired by the radiation detector 5 on the side close to the subject H. It should be noted that the second radiation image G2 may be used. In addition, regardless of which image is used, a low-frequency image representing a low-frequency component of the image may be derived, to use the low-frequency image to derive the body thickness.

In the present embodiment, the body thickness acquisition unit 22 derives the body thickness of the subject H by assuming that a brightness distribution of the first radiation image G1 coincides with the body thickness distribution of the subject H, and converting the pixel value of the first radiation image G1 into the thickness by using an attenuation coefficient of the soft part of the subject H. Instead of the above, the body thickness acquisition unit 22 may measure the thickness of the subject H by using a sensor or the like. Also, the body thickness acquisition unit 22 may derive the body thickness by approximating the body thickness of the subject H with a model such as a cube or an elliptical column. The body thickness acquisition unit 22 may derive the body thickness of the subject H by any method such as a method described in JP2015-043959A.

The component thickness derivation unit 23 derives the thicknesses of the first component to the third component included in the subject H, that is, the thickness of the fat, the thickness of the muscle, and the thickness of the bone part, by using the body thickness derived by the body thickness acquisition unit 22, the first radiation image G1, and the second radiation image G2.

First, the component thickness derivation unit 23 derives a first attenuation image CL and a second attenuation image CH representing the attenuation amount of the radiation by the subject H from each of the first radiation image G1 and the second radiation image G2.

A pixel value of the first attenuation image CL represents the attenuation amount of the low-energy radiation due to the subject H, and a pixel value of the second attenuation image CH represents the attenuation amount of the high-energy radiation due to the subject H. The component thickness derivation unit 23 derives the first attenuation image CL and the second attenuation image CH from the first radiation image G1 and the second radiation image G2 by Expression (1) and Expression (2). In the expression (1), Gd1 is the pixel value of the direct radiation region in the first radiation image G1, and, in the expression (2), Gd2 is the pixel value of the direct radiation region in the second radiation image G2.

$$CL(x,y) = Gd1 - G1(x,y) \quad (1)$$

$$CH(x,y) = Gd2 - G2(x,y) \quad (2)$$

The attenuation amount of the radiation by the subject H is determined depending on the thickness of the fat, the thickness of the muscle, the thickness of the bone part, and the radiation quality (high-energy or low-energy). Therefore, in a case in which the attenuation coefficient representing an attenuation rate per unit thickness is $\mu$, attenuation amounts CL0 and CH0 of the radiation at each pixel position in each of the low-energy image and the high-energy image can be represented by Expression (3) and Expression (4). In Expression (3) and Expression (4), tf is a thickness of the fat, tm is a thickness of the muscle, tb is a thickness of the bone part, $\mu$Lf is a fat attenuation coefficient of the low-energy radiation, $\mu$Lm is a muscle attenuation coefficient of the low-energy radiation, $\mu$Lb is a bone part attenuation coefficient of the low-energy radiation, $\mu$Hf is a fat attenuation coefficient of the high-energy radiation, $\mu$Hm is a muscle attenuation coefficient of the high-energy radiation, and $\mu$Hb is a bone part attenuation coefficient of the high-energy radiation. The attenuation coefficient represents an attenuation rate of radiation per unit thickness.

$$CL0 = \mu Lf(tf,tm,tb) \times tf + \mu Lm(tf,tm,tb) \times tm + \mu Lb(tf,tm,tb) \times tb \quad (3)$$

$$CH0 = \mu Hf(tf,tm,tb) \times tf + \mu Hm(tf,tm,tb) \times tm + \mu Hb(tf,tm,tb) \times tb \quad (4)$$

In Expression (3) and Expression (4), the attenuation amount CL0 of the low-energy image corresponds to the pixel value of the first attenuation image CL, and the attenuation amount CH0 of the high-energy image corresponds to the pixel value of the second attenuation image CH. Expression (3) and Expression (4) represent a relationship between the first attenuation image CL and the second attenuation image CH in each pixel, but (x,y) representing the pixel position is omitted. In Expression (5) to Expression (11) shown later, (x,y) representing the pixel position is also omitted.

In Expression (3) and Expression (4), the variables are the thickness tf of the fat, the thickness tm of the muscle, and the thickness tb of the bone part. Since there are three variables, the thickness tf of the fat, the thickness tm of the muscle, and the thickness tb of the bone part cannot be derived only by two Expressions (3) and (4). In the present embodiment, the body thickness acquisition unit 22 acquires the body thickness of the subject H. A relationship between the body thickness T and the thickness tf of the fat, the thickness tm of the muscle, and the thickness tb of the bone part is represented by Expression (5). Expression (5) is modified for the thickness tb of the bone part, to obtain Expression (6).

$$T = tb + tm + tf \quad (5)$$

$$tb = T - tm - tf \quad (6)$$

Since the attenuation amount CL0 of the low-energy image corresponds to the pixel value of the first attenuation image CL and the attenuation amount CH0 of the high-energy image corresponds to the pixel value of the second attenuation image CH, in a case in which Expression (6) is substituted into Expression (3) and Expression (4), Expression (7) and Expression (8) are obtained.

$$CL = \mu Lf(tf,tm,T - tm - tf) \times tf + \mu Lm(tf,tm,T - tm - tf) \times tm + \mu Lb(tf,tm,T - tm - tf) \times (T - tm - tf) \qquad (7)$$

$$CH = \mu Hf(tf,tm,T - tm - tf) \times tf + \mu Hm(tf,tm,T - tm - tf) \times tm + \mu Hb(tf,tm,T - tm - tf) \times (T - tm - tf) \qquad (8)$$

In the present embodiment, since the body thickness T is acquired by the body thickness acquisition unit 22, the variables in Expression (7) and Expression (8) are two variables of the thickness tf of the fat and the thickness tm of the muscle. Therefore, the thickness tf of the fat and the thickness tm of the muscle can be derived by solving Expression (7) and Expression (8) with the thickness tf of the fat and the thickness tm of the muscle as variables. Further, the thickness tb of the bone part can be derived by Expression (6) from the body thickness T, the derived thickness tf of the fat, and the derived thickness tm of the muscle.

For solving Expression (7) and Expression (8), the fat attenuation coefficients $\mu Lf$ and $\mu Hf$, the muscle attenuation coefficients $\mu Lm$ and $\mu Hm$, and the bone part attenuation coefficients $\mu Lb$ and $\mu Hb$ for each of the low-energy radiation and the high-energy radiation are required.

Here, in a procedure of the radiation transmitted through the subject H, a low-energy component of the radiation is absorbed by the subject H, and beam hardening occurs in which the radiation energy is increased. Therefore, in the present embodiment, the muscle attenuation coefficients $\mu Lm$ and $\mu Hm$, the fat attenuation coefficients $\mu Lf$ and $\mu Hf$, and the bone part attenuation coefficients $\mu Lb$ and $\mu Hb$ are derived in consideration of beam hardening in advance, and then stored in the storage 13.

In the human body, the fat and the muscle are mixed, and the bone part is present in the human body, so that the radiation is transmitted through the fat, the muscle, and the bone part in a complicated order in accordance with the overlap thereof inside the human body. On the other hand, in a case in which monochromatic radiation is considered, the monochromatic radiation does not have a change in spectrum, so that the fat, the muscle, and the bone part have a constant attenuation coefficient. In a case in which the total thicknesses of the fat, the muscle, and the bone part are the same, the radiation dose after the transmission through the subject is always the same regardless of the order in which the radiation is transmitted through the respective components in the subject.

Radiation actually emitted to the subject is continuous radiation having a wide spectrum, but is a collection of the monochromatic radiation. Therefore, even in a case of the continuous radiation, in a case in which the total thicknesses of the fat, the muscle, and the bone part are the same, the radiation dose after the radiation is transmitted through the subject is always the same regardless of the order in which the components are transmitted.

In the present embodiment, the thickness tf of the fat, the thickness tm of the muscle, and the thickness tb of the bone part are required to derive a component image described below, and the information on how the fat, the muscle, and the bone part overlap each other is not required. Therefore, in the present embodiment, the attenuation coefficient is derived by using three types of models, that is, a model consisting of only the fat, a simple model in which the radiation is first transmitted through the fat and then transmitted through the muscle, and a simple model in which the radiation is first transmitted through the fat, then transmitted through the muscle, and then transmitted through the bone. It should be noted that, in the model, the fat, the muscle, and the bone part are objects having the attenuation coefficients corresponding to the fat, the muscle, and the bone part, respectively. In addition, the order of the components in the model is not limited to the fat, the muscle, and the bone part, and any order can be adopted.

First, for the fat attenuation coefficient, a model consisting of only the fat, that is, a model consisting of only an object having the attenuation coefficient corresponding to the fat is used, and the low-energy image and the high-energy image are derived by irradiating the model with the low-energy radiation and the high-energy radiation while changing the thickness of the fat. Then, a ratio of the pixel value of each pixel of the low-energy image and the high-energy image to an image (hereinafter, referred to as a direct image) acquired by the low-energy radiation and the high-energy radiation in a case in which the fat is not present is derived, and the ratio is further divided by the thickness of the fat to derive the fat attenuation coefficients $\mu Lf$ and $\mu Hf$ for various thicknesses of the fat.

For the muscle attenuation coefficient, a model consisting of the fat and the muscle, that is, a model consisting of an object having the attenuation coefficient corresponding to each of the fat and the muscle is used, and the low-energy radiation and the high-energy radiation are respectively emitted to the model while changing the thicknesses of the fat and the muscle, to derive the low-energy image and the high-energy image. A ratio of the pixel value of each pixel of the low-energy image and the high-energy image to the pixel value of the direct image is derived, and the ratio is further divided by the thickness of the muscle to derive the muscle attenuation coefficients $\mu Lm$ and $\mu Hm$ for various thicknesses of the muscle. In the derivation of the muscle attenuation coefficients $\mu Lm$ and $\mu Hm$, the thickness of the fat is also taken into consideration, so that the muscle attenuation coefficients $\mu Lm$ and $\mu Hm$ are obtained in consideration of the beam hardening of the fat.

For the bone part attenuation coefficient, a model consisting of the fat, the muscle, and the bone part, that is, a model consisting of an object having the attenuation coefficient corresponding to each of the fat, the muscle, and the bone part is used, and the low-energy radiation and the high-energy radiation are respectively emitted to the model while changing the thicknesses of the fat, the muscle, and the bone part, to derive the low-energy image and the high-energy image. A ratio of the pixel value of each pixel of the low-energy image and the high-energy image to the pixel value of the direct image is derived, and the ratio is further divided by the thickness of the bone part to derive the bone part attenuation coefficients $\mu Lb$ and $\mu Hb$ for various thicknesses of the bone part. In the derivation of the bone part attenuation coefficients $\mu Lb$ and $\mu Hb$, the thicknesses of the fat and the muscle are also taken into consideration, so that the bone part attenuation coefficients $\mu Lb$ and $\mu Hb$ are obtained in consideration of the beam hardening of the fat and the muscle.

For example, in a case in which the bone part attenuation coefficient $\mu Lb1(tf1,tm1,tb1)$ in a case of $tf = tf1$, $tm = tm1$, and $tb = tb1$ is derived, assuming that the pixel value of the acquired low-energy component image is CL1 and the pixel value of the direct image is CLd, the bone part attenuation coefficient $\mu Lb1(tf1,tm1,tb1)$ is derived by Expression (9).

$$\mu Lb1(tf1,tm1,tb1) = CL1/CLd \ (9)$$

Here, the thicknesses of the three types of substances included in the model are discrete. Therefore, for the three types of substances, the attenuation coefficients derived for various thicknesses can be interpolated to derive a relationship between the thickness and the attenuation coefficient of each component.

It should be noted that the fat attenuation coefficients $\mu Lf$ and $\mu Hf$ derived as described above are represented by a one-dimensional look-up table that depends on only the thickness of the fat. The muscle attenuation coefficients $\mu Lm$ and $\mu Hm$ are represented by a two-dimensional look-up table that depends on only the thicknesses of the fat and the muscle. The bone part attenuation coefficients $\mu Lb$ and $\mu Hb$ are represented by a three-dimensional look-up table that depends on the thicknesses of the fat, the muscle, and the bone part.

As described above, the component thickness derivation unit 23 derives the thickness tf of the fat and the thickness tm of the muscle by solving Expression (7) and Expression (8) with the thickness tf of the fat and the thickness tm of the muscle as variables. It should be noted that the thickness tf of the fat and the thickness tm of the muscle, which are derived, are derived for each pixel of the first attenuation image CL and the second attenuation image CH, but, in the following description, (x,y) representing the pixel position will be omitted.

The component thickness derivation unit 23 first calculates the thickness tm of the muscle in a case in which the thickness tf of the fat is set to zero by Expression (8). In a case in which $tf = 0$ and $tm = tm0$, $CH = \mu Hf(0, tm0, T - tm0 - 0) \times 0 + \mu Hm(0, tm0, T - tm0 - 0) \times tm0 + \mu Hb(0, tm0, T - tm0 - 0) \times (T - tm0 - 0)$, and thus tm0 is calculated by Expression (10). In addition, the component thickness derivation unit 23 calculates the thickness tf0 of the fat in a case in which the thickness tm of the muscle is set to zero, by using Expression (8). In a case in which $tf = tf0$ and $tm = 0$, $CH = \mu Hf(tf0, 0, T - 0 - tf0) \times tf0 + \mu Hm(tf0, 0, T - 0 - tf0) \times 0 + \mu Hb(tf0, 0, T - 0 - tf0) \times (T - 0 - tf0)$, and thus tf0 is calculated by Expression (11).

$$tm0 = CH/(\mu Hm(0,tm0,T - tm0 - 0) \times tm0 + \mu Hb(0,tm0,T - tm0) \times (T - tm0)) \ (10)$$

$$tf0 = CH/(\mu Hf(tf0,0,T - 0 - tf0) \times tf0 + \mu Hb(tf0,0,T - tf0) \times (T - tf0)) \ (11)$$

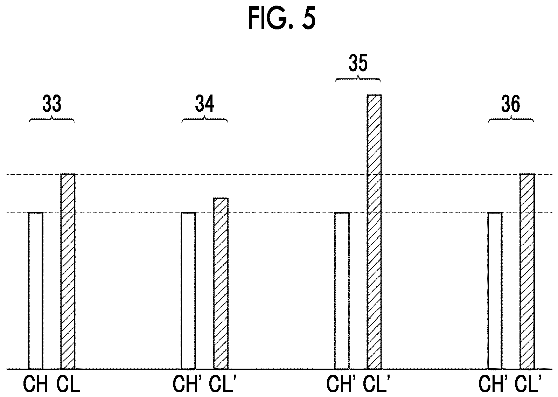

Fig. 5 is a diagram showing a relationship between the attenuation amounts in accordance with the thickness of the muscle and the thickness of the fat. In Fig. 5, an attenuation amount 33 indicates the attenuation amount CL which is the pixel value of the low-energy image and the attenuation amount CH which is the pixel value of the high-energy image which are derived by actually imaging the subject. Here, the attenuation amount of the low-energy image and the attenuation amount of the high-energy image are larger as the density of the composition is higher. Therefore, the composition in a case in which $tm = 0$ and $tf = tf0$ has a lower density than the composition based on the actual thickness of the muscle and the actual thickness of the fat. Therefore, in a case in which $tm = 0$ and $tf = tf0$, the pixel value, that is, the attenuation amount of the first attenuation image (here, a provisional first attenuation image CL') derived by Expression (7) is less than the pixel value of the first attenuation image CL derived from the actual thickness of the muscle and the actual thickness of the fat as shown in an attenuation amount 34 of Fig. 5 (that is, CL > CL').

On the other hand, the composition in a case in which $tm = tm0$ and $tf = 0$ has a higher density than the composition based on the actual thickness of the muscle and the actual thickness of the fat. Therefore, in a case in which $tm = tm0$ and $tf = 0$, the pixel value, that is, the attenuation amount of the provisional first attenuation image CL' derived by Expression (7) is greater than the pixel value of the first attenuation image CL derived from the actual thickness of the muscle and the actual thickness of the fat as shown in an attenuation amount 35 of Fig. 5 (that is, CL < CL').

It should be noted that the pixel value, that is, the attenuation amount of the provisional first attenuation image CL' derived by Expression (7) by using the actual thickness of the muscle and the actual thickness of the fat is the same as the pixel value of the first attenuation image CL as shown in an attenuation amount 36 of Fig. 5. By using this fact, the component thickness derivation unit 23 derives the thickness tm of the muscle and the thickness tf of the fat as follows.

Step S1

First, in Expression (8), a provisional thickness tfk of the fat is set. It should be noted that zero is used as an initial value of the provisional thickness tmk of the muscle.

Step S2

Then, the provisional first attenuation image CL' is calculated by Expression (7) using the provisional thickness tfk of the fat, the provisional thickness tmk of the muscle, the fat attenuation coefficient $\mu Lf$, the muscle attenuation coefficient $\mu Lm$, and the bone part attenuation coefficient $\mu Lb$ for the low-energy radiation. As the fat attenuation coefficient $\mu Lf$, the muscle attenuation coefficient $\mu Lm$, and the bone part attenuation coefficient $\mu Lb$, the values in accordance with the provisional thickness tfk of the fat, the provisional thickness tmk of the muscle, and the provisional thickness of the bone part (that is, T - tfk - tmk) are used.

Step S3

Next, the difference value $\Delta CL$ between the provisional first attenuation image CL' and the first attenuation image CL is calculated. The provisional thickness tmk of the muscle is updated on the assumption that the difference value $\Delta CL$ is the pixel value corresponding to the amount of the radiation attenuated by the bone.

Step S4

Next, the provisional second attenuation image CH' is calculated by Expression (8) using the updated thickness tmk of the muscle and thickness tfk of the fat.

Step S5

Next, the difference value $\Delta CH$ between the provisional second attenuation image CH' and the second attenuation image CH is calculated. The provisional thickness tfk of the fat is updated on the assumption that the difference value $\Delta CH$ is the pixel value corresponding to the amount of the radiation attenuated by the fat.

Then, the thickness tf of the fat and the thickness tm of the muscle are derived by repeating the processing of steps S1 to S5 until the absolute values of the difference values $\Delta CL$ and $\Delta CH$ are less than a predetermined threshold value. It should be noted that the thickness tf of the fat and the thickness tm of the muscle may be derived by repeating the processing of steps S1 to S5 a predetermined number of times.

The image derivation unit 24 derives the first to third component images, that is, the fat image Gf, the muscle image Gm, and the bone part image Gb, based on the thickness tf of the fat and the thickness tm of the muscle that are derived by the component thickness derivation unit 23 and the thickness tb of the bone part derived by Expression (6). The fat image Gf has a pixel value having a size in accordance with the thickness tf of the fat. The muscle image Gm has a pixel value having a size in accordance with the thickness tm of the muscle. The bone part image Gb has a pixel value having a size in accordance with the thickness tb (that is, T - tf - tm) of the bone part.

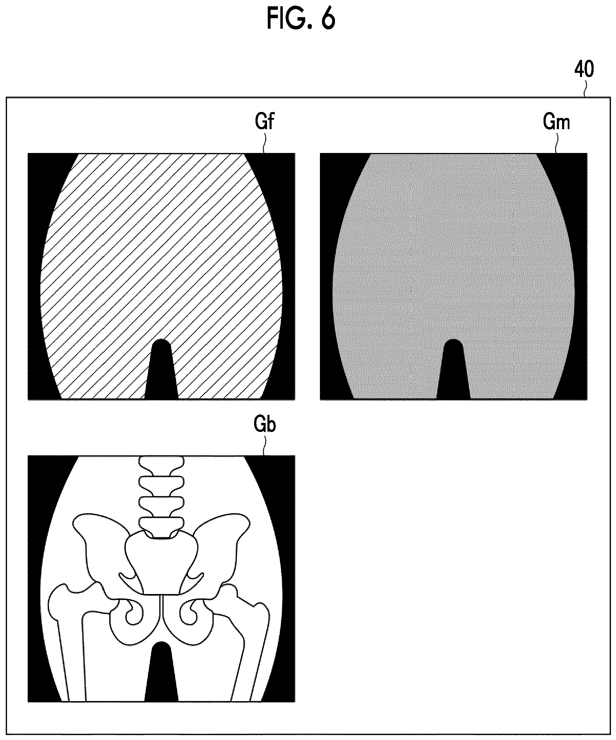

The display controller 25 displays the fat image Gf, the muscle image Gm, and the bone part image Gb that are derived. Fig. 6 is a diagram showing a display screen 40 of the fat image Gf, the muscle image Gm, and the bone part image Gb.

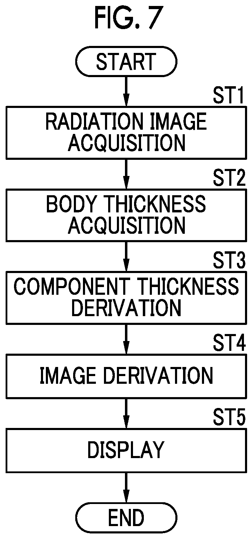

Hereinafter, the processing performed in the present embodiment will be described. Fig. 7 is a flowchart showing the processing performed in the present embodiment. The image acquisition unit 21 causes the imaging apparatus 1 to perform the energy subtraction imaging of the subject H to acquire the first and second radiation images G1 and G2 (radiation image acquisition: step ST1). Then, the body thickness acquisition unit 22 acquires the body thickness T of the subject H (step ST2).

Then, the component thickness derivation unit 23 derives the thickness tf of the fat, the thickness tm of the muscle, and the thickness tb of the bone part by using the body thickness T, the first radiation image G1, and the second radiation image G2 (component thickness derivation: step ST3). Next, the image derivation unit 24 derives the fat image Gf, the muscle image Gm, and the bone part image Gb based on the thickness tf of the fat, the thickness tm of the muscle, and the thickness tb of the bone part (component image derivation: step ST4). Then, the display controller 25 displays the fat image Gf, the muscle image Gm, and the bone part image Gb (step ST5), and the processing ends.

As described above, in the present embodiment, the thickness tf of the fat, the thickness tm of the muscle, and the thickness tb of the bone part are derived by using the body thickness T, the first radiation image G1, and the second radiation image G2, and the fat image Gf, the muscle image Gm, and the bone part image Gb are derived based on the thickness tf of the fat, the thickness tm of the muscle, and the thickness tb of the bone part. Therefore, it is possible to acquire the fat image Gf, the muscle image Gm, and the bone part image Gb for the subject H only by acquiring two images having different energy distributions, which are the first and second radiation images G1 and G2. Therefore, the fat component, the muscle component, and the bone part component in the subject H can be accurately separated by using two images having different energy distributions, which are the first and second radiation images G1 and G2.

It should be noted that, in the embodiment described above, the images of three components of the fat component, the muscle component, and the bone part component in the subject H are derived by using the first and second radiation images G1 and G2, but the present disclosure is not limited to this. The first to (n - 1)th radiation images may be acquired by n - 1 types of radiation having different energy distributions, and the first to nth component images may be derived by using the body thickness and the first to (n - 1)th radiation images. For example, the thickness of the component of the artificial object in the subject H may be derived in addition to the fat component, the muscle component, and the bone part component in the subject H from the body thickness of the subject H and three radiation images having different energy distributions by using the three radiation images, to derive four component images including the fat image, the muscle image, the bone part image, and the artificial object image. Examples of the artificial object include a screw for fixing a fracture portion, a stent disposed in a blood vessel, a silicone disposed in a breast, and a contrast agent.

Also, in the embodiment described above, the first and second radiation images G1 and G2 are acquired by the one-shot method, but the present disclosure is not limited to this. The first and second radiation images G1 and G2 may be acquired by a so-called two-shot method in which the imaging is performed twice by using only one radiation detector. In a case of the two-shot method, a position of the subject H included in the first radiation image G1 and the second radiation image G2 may shift due to a body movement of the subject H. Therefore, in the first radiation image G1 and the second radiation image G2, it is preferable to perform the processing according to the present embodiment after registration of the subject is performed.

In addition, in the embodiment described above, the radiation image acquired in the system that images the subject H using the first and second radiation detectors 5 and 6 is used, but it goes without saying that the technology of the present disclosure can be applied even in a case in which the first and second radiation images G1 and G2 are acquired using an accumulative phosphor sheet instead of the radiation detector. In this case, the first and second radiation images G1 and G2 need only be acquired by stacking two accumulative phosphor sheets, emitting the radiation transmitted through the subject H, accumulating and recording radiation image information of the subject H in each of the accumulative phosphor sheets, and photoelectrically reading the radiation image information from each of the accumulative phosphor sheets. It should be noted that the two-shot method may also be used in a case in which the first and second radiation images G1 and G2 are acquired by using the accumulative phosphor sheet.

In addition, the radiation in the embodiment described above is not particularly limited, and $\alpha$-rays or $\gamma$-rays can be used in addition to X-rays.

In addition, in the embodiment described above, various processors shown below can be used as the hardware structure of processing units that execute various types of processing, such as the image acquisition unit 21, the body thickness acquisition unit 22, the component thickness derivation unit 23, the image derivation unit 24, and the display controller 25. As described above, the various processors include, in addition to the CPU that is a general-purpose processor which executes software (program) and functions as various processing units, a programmable logic device (PLD) that is a processor whose circuit configuration can be changed after manufacture, such as a field programmable gate array (FPGA), and a dedicated electrical circuit that is a processor having a circuit configuration which is designed for exclusive use in order to execute specific processing, such as an application specific integrated circuit (ASIC).

One processing unit may be configured by one of these various processors, or may be configured by a combination of two or more processors of the same type or different types (for example, a combination of a plurality of FPGAs or a combination of the CPU and the FPGA). In addition, a plurality of the processing units may be configured by one processor.

As an example of configuring the plurality of processing units by one processor, first, as represented by a computer of a client, a server, and the like there is an aspect in which one processor is configured by a combination of one or more CPUs and software and this processor functions as a plurality of processing units. Second, as represented by a system on chip (SoC) or the like, there is an aspect of using a processor that implements the function of the entire system including the plurality of processing units by one integrated circuit (IC) chip. In this way, as the hardware structure, the various processing units are configured by using one or more of the various processors described above.

Further, as the hardware structures of these various processors, more specifically, it is possible to use an electrical circuit (circuitry) in which circuit elements, such as semiconductor elements, are combined.

Hereinafter, the supplementary notes of the present disclosure will be described.

Supplementary Note 1

A radiation image processing device comprising: at least one processor, in which the processor is configured to: acquire first to (n - 1)th (n ≥ 3) radiation images acquired by imaging a subject, which includes first to nth components each consisting of a single composition, with n - 1 types of radiation having different energy distributions; acquire a body thickness of the subject; derive thicknesses of the first to nth components by using the body thickness and the first to (n - 1) th radiation images; and derive first to nth component images in which the first to nth components are enhanced, respectively, based on the thicknesses of the first to nth components.

Supplementary Note 2

The radiation image processing device according to supplementary note 1, in which the processor is configured to acquire the body thickness by deriving the body thickness of the subject based on at least one of the first to (n - 1)th radiation images.

Supplementary Note 3

The radiation image processing device according to supplementary note 1 or 2, in which the processor is configured to: acquire a first radiation image and a second radiation image that are acquired by imaging the subject with two types of radiation having different energy components; derive the body thickness of the subject based on at least one of the first radiation image or the second radiation image; derive thicknesses of a first component, a second component, and a third component of the subject by using the body thickness, the first radiation image, and the second radiation image; and derive a first component image, a second component image, and a third component image in which the first component, the second component, and the third component are enhanced, respectively, based on the thicknesses of the first component, the second component, and the third component.

Supplementary Note 4

The radiation image processing device according to supplementary note 3, in which the first component, the second component, and the third component are fat, muscle, and a bone, respectively.

Supplementary Note 5

A radiation image processing method executed by a computer, the radiation image processing method comprising: acquiring first to (n - 1)th (n ≥ 3) radiation images acquired by imaging a subject, which includes first to nth components each consisting of a single composition, with n - 1 types of radiation having different energy distributions; acquiring a body thickness of the subject; deriving thicknesses of the first to nth components by using the body thickness and the first to (n - 1)th radiation images; and deriving first to nth component images in which the first to nth components are enhanced, respectively, based on the thicknesses of the first to nth components.

Supplementary Note 6

A radiation image processing program causing a computer to execute: a procedure of acquiring first to (n - 1)th (n ≥ 3) radiation images acquired by imaging a subject, which includes first to nth components each consisting of a single composition, with n - 1 types of radiation having different energy distributions; a procedure of acquiring a body thickness of the subject; a procedure of deriving thicknesses of the first to nth components by using the body thickness and the first to (n - 1)th radiation images; and a procedure of deriving first to nth component images in which the first to nth components are enhanced, respectively, based on the thicknesses of the first to nth components.

Explanation of References

1: imaging apparatus
3: radiation source
5, 6: radiation detector
7: radiation energy conversion filter
10: image processing device
11: CPU
12: radiation image processing program
13: storage
14: display

15: input device
16: memory
17: network I/F
18: bus
21: image acquisition unit
22: body thickness acquisition unit
23: component thickness derivation unit
24: image derivation unit
25: display controller
33 to 36: attenuation amount
40: display screen
Gf: fat image
Gm: muscle image
Gb: bone part image
H: subject

**Claims**

1. A radiation image processing device comprising:

at least one processor,
wherein the processor is configured to:

acquire first to (n - 1)th (n ≥ 3) radiation images acquired by imaging a subject, which includes first to nth components each consisting of a single composition, with n - 1 types of radiation having different energy distributions;
acquire a body thickness of the subject;
derive thicknesses of the first to nth components by using the body thickness and the first to (n - 1)th radiation images; and
derive first to nth component images in which the first to nth components are enhanced, respectively, based on the thicknesses of the first to nth components.

2. The radiation image processing device according to claim 1,
wherein the processor is configured to acquire the body thickness by deriving the body thickness of the subject based on at least one of the first to (n - 1)th radiation images.

3. The radiation image processing device according to claim 1 or 2,
wherein the processor is configured to:

acquire a first radiation image and a second radiation image that are acquired by imaging the subject with two types of radiation having different energy components;
derive the body thickness of the subject based on at least one of the first radiation image or the second radiation image;
derive thicknesses of a first component, a second component, and a third component of the subject by using the body thickness, the first radiation image, and the second radiation image; and
derive a first component image, a second component image, and a third component image in which the first component, the second component, and the third component are enhanced, respectively, based on the thicknesses of the first component, the second component, and the third component.

4. The radiation image processing device according to claim 3,
wherein the first component, the second component, and the third component are fat, muscle, and a bone, respectively.

5. A radiation image processing method executed by a computer, the radiation image processing method comprising:

acquiring first to (n - 1)th (n ≥ 3) radiation images acquired by imaging a subject, which includes first to nth components each consisting of a single composition, with n - 1 types of radiation having different energy distributions;
acquiring a body thickness of the subject;
deriving thicknesses of the first to nth components by using the body thickness and the first to (n - 1)th radiation images; and
deriving first to nth component images in which the first to nth components are enhanced, respectively, based on the thicknesses of the first to nth components.

6. A computer-readable storage medium that stores a radiation image processing program causing a computer to execute:

a procedure of acquiring first to (n - 1)th ($n \geq 3$) radiation images acquired by imaging a subject, which includes first to nth components each consisting of a single composition, with n - 1 types of radiation having different energy distributions;
a procedure of acquiring a body thickness of the subject;
a procedure of deriving thicknesses of the first to nth components by using the body thickness and the first to (n - 1)th radiation images; and
a procedure of deriving first to nth component images in which the first to nth components are enhanced, respectively, based on the thicknesses of the first to nth components.

# FIG. 1

# FIG. 2

# FIG. 3

| | |
|---|---|
| RADIATION IMAGE PROCESSING DEVICE | ~10 |
| IMAGE ACQUISITION UNIT | ~21 |
| BODY THICKNESS ACQUISITION UNIT | ~22 |
| COMPONENT THICKNESS DERIVATION UNIT | ~23 |
| IMAGE DERIVATION UNIT | ~24 |
| DISPLAY CONTROLLER | ~25 |

# FIG. 4

G1,G2

FIG. 5

# FIG. 6

## FIG. 7

```
         ┌──────────┐
         │  START   │
         └──────────┘
              │
              ▼                ST1
    ┌─────────────────────┐
    │  RADIATION IMAGE    │
    │    ACQUISITION      │
    └─────────────────────┘
              │
              ▼                ST2
    ┌─────────────────────┐
    │   BODY THICKNESS    │
    │    ACQUISITION      │
    └─────────────────────┘
              │
              ▼                ST3
    ┌─────────────────────┐
    │ COMPONENT THICKNESS │
    │    DERIVATION       │
    └─────────────────────┘
              │
              ▼                ST4
    ┌─────────────────────┐
    │  IMAGE DERIVATION   │
    └─────────────────────┘
              │
              ▼                ST5
    ┌─────────────────────┐
    │      DISPLAY        │
    └─────────────────────┘
              │
              ▼
         ┌──────────┐
         │   END    │
         └──────────┘
```

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/153970 A1 (IWASHITA ATSUSHI [JP] ET AL) 18 May 2023 (2023-05-18)<br>* paragraph [0033] - paragraph [0034] *<br>* paragraph [0064] - paragraph [0099] *<br>* paragraph [0101] - paragraph [0124] *<br>* paragraph [0140] - paragraph [0149] *<br>* paragraph [0174] - paragraph [0176] *<br>* figures 1,5-18 * | 1-6 | INV.<br>A61B6/00<br>A61B6/50<br>G01N23/04 |
| X | JP 2010 005252 A (FUJIFILM CORP) 14 January 2010 (2010-01-14)<br>* paragraph [0009] *<br>* paragraph [0023] - paragraph [0057] * | 1,2,5,6 | |
| X | US 2023/134187 A1 (KAWAMURA TAKAHIRO [JP]) 4 May 2023 (2023-05-04) | 1,2,5,6 | |
| A | * paragraph [0011] - paragraph [0015] *<br>* figures 4-11 *<br>* claim 5 *<br>* paragraph [0059] - paragraph [0062] * | 3,4 | |
| X | US 2023/404510 A1 (TAKI TOMOKO [JP]) 21 December 2023 (2023-12-21) | 1,2,5,6 | TECHNICAL FIELDS SEARCHED (IPC) |
| A | * claims 1-4 *<br>* figure 12 *<br>* paragraph [0073] - paragraph [0078] *<br>* paragraph [0086] - paragraph [0096] * | 3,4 | A61B |
| X | US 2022/167935 A1 (IWASHITA ATSUSHI [JP] ET AL) 2 June 2022 (2022-06-02) | 1,2,5,6 | |
| A | * figures 5-10 *<br>* paragraph [0061] - paragraph [0102] *<br>* paragraph [0122] * | 3,4 | |

The present search report has been drawn up for all claims

| Place of search | Date of completion of the search | Examiner |
|---|---|---|
| Munich | 14 July 2025 | De la Hera, Germán |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

...................................................................

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 25 16 2809

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2025

| Patent document cited in search report | Publication date | Patent family member(s) | | Publication date |
|---|---|---|---|---|
| US 2023153970 A1 | 18-05-2023 | JP | 2022056084 A | 08-04-2022 |
| | | US | 2023153970 A1 | 18-05-2023 |
| | | WO | 2022071024 A1 | 07-04-2022 |
| JP 2010005252 A | 14-01-2010 | JP | 5124363 B2 | 23-01-2013 |
| | | JP | 2010005252 A | 14-01-2010 |
| US 2023134187 A1 | 04-05-2023 | JP | 2023068496 A | 17-05-2023 |
| | | US | 2023134187 A1 | 04-05-2023 |
| US 2023404510 A1 | 21-12-2023 | CN | 117257331 A | 22-12-2023 |
| | | JP | 2024000885 A | 09-01-2024 |
| | | US | 2023404510 A1 | 21-12-2023 |
| US 2022167935 A1 | 02-06-2022 | EP | 4014874 A1 | 22-06-2022 |
| | | JP | 7373323 B2 | 02-11-2023 |
| | | JP | 2021037031 A | 11-03-2021 |
| | | US | 2022167935 A1 | 02-06-2022 |
| | | WO | 2021044754 A1 | 11-03-2021 |

EPO FORM P0459

For more details about this annex : see Official Journal of the European Patent Office, No. 12/82

**EP 4 616 807 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023047911 A
- JP 2022056084 A
- JP 2015043959 A